# EUROPEAN PATENT APPLICATION

(11) **EP 4 276 166 A1**
(43) Date of publication of application: **15.11.2023**
(21) Application number: 22736774.5
(22) Date of filing: 06.01.2022
(51) Int. Cl.: C12M 1/00, C12M 1/04, C12M 3/00

(54) **CELL CULTURE DEVICE AND PARALLEL FILTER CONNECTOR**

(30) Priority: 11.01.2021 JP 2021002489
(71) Applicant: JCR Pharmaceuticals Co., Ltd., Ashiya-shi, Hyogo 659-0021 (JP)
(72) Inventor: WATANABE, Shunsuke, Kobe-shi, Hyogo 651-2241 (JP); KUSANO, Soichiro, Tokyo 100-0013 (JP)
(74) Representative: Isarpatent
(86) International application number: PCT/JP2022/000286
(87) International publication number: WO 2022/149606

(57) **Abstract**

Disclosed are cell culture vessels capable of preventing the deformation of the culture vessels in a manipulation of taking in/out a cell or replacing a culture medium and preventing over-time increase in the concentration of carbon dioxide or and temporal decrease of pH of the culture medium and over-time temporal decrease of pH of the culture medium in cell culture as advantageous effects, and further parallel filter connectors for configuring such cell culture vessels. The cell culture vessels have two types of filters in parallel so as to partition a gas phase inside the cell culture vessel and the outer air, and the parallel filter connectors for configuring the cell culture vessels have an opening to be connected to the body of the cell culture vessel, and two openings capable to be installed with each one of the two types of different filters.

## Description

### TECHNICAL FIELD

The present invention relates to a cell culture vessel (hereinafter, also simply referred to as a "culture vessel") that is a container in which a cell and a liquid culture medium are to be placed and cell culture is performed, in particular, a cell culture vessel in which two different types of filters are parallelly installed at the interface between the internal and external air phases, and relates to a parallel-filter connector for attaching such filters in parallel to the body of the cell culture vessel.

### BACKGROUND ART

In order to produce biological medicine such as a vaccine, a recombinant protein pharmaceutical product, and a cellular medicine, in general, large-scale cell culture is performed. There are two types of cells to be cultured, which includes cells that can be cultured in a suspended state in a liquid culture medium (hereinafter, also referred to as a "floating cell") and cells that is practically required to be cultured in an adhered state to the surface of a support body functioning as a scaffold (hereinafter, also referred to as an "adherent cell"). In the case of the floating cell, it is possible to perform the large-scale culture in a relatively small occupied area by performing shake culture using a flask or spinner culture using an agitation blade or the like installed inside the culture vessel. On the other hand, in the culture of the adherent cell, it is not practical to agitate the culture medium. Accordingly, in the culture of the adherent cell, in order to sufficiently supply oxygen required for the survival and proliferation of the cell and to remove the produced carbon dioxide, it is necessary to increase a surface area per the volume of the culture medium and to decrease the liquid depth of the culture medium such that gas exchange between the gas phase and the liquid phase is to be promoted.

In culturing the adherent cell, a process of medium exchange is generally carried out in which the used liquid medium is removed and a fresh medium is added at proper intervals. Accordingly, in the culture vessel for the adherent cell, an opening for performing the taking-in/out of the cell, the culture medium replacement, and the like is provided. Such an opening is closed with a cap during a culture period such that the inside of the culture vessel is not contaminated. However, if the gas phase inside the culture vessel is completely cut off from the outer air, the composition of the gas phase in the culture vessel is gradually changed in accordance with the metabolism of the cell, and therefore, it is necessary that a sufficient amount of gas exchange must be assured between the inside and outside of the culture vessel even when the opening is closed with the cap so as to prevent such change. Accordingly, a cap including a filter (a membrane filter) that enables the gas phase to pass through to some extent is attached to the culture vessel, and the gas exchange between the gas phase inside the culture vessel and the gas phase outside the culture vessel is performed through the filter.

In culturing adherent cells, the number of cells that can be cultured has a positive correlation with the area of the surface in the culture vessel to which the cell is capable of adhering. Accordingly, in order to efficiently perform the large-scale culture of the adherent cell, it is necessary to increase the area of the surface in the culture vessel to which the cell is capable of adhering. For this purpose, a multi-layer culture vessel has been developed (CELLSTACK: Registered Trademark) which is a cell culture vessel with integrated multiple trays having supporting surfaces which allow cell adhesion (Patent Document 1). A culture vessel of this type is in the form of stacked layers of those chambers and has a common opening with a cap which is removed when loading and removing cells on each tray and at medium exchange, and a common opening for exchange between the internal and external gas phases (to which a cap with membrane filter is to be attached), on the upper part of the stack. In performing a cell culture, a medium is added to the culture vessel in such a manner that the flat upper surface of the support provided in each tray is submerged in the liquid medium while assuring a gas phase. As described above, the cell adheres to the surface of the support body in each of the trays, and thus, the survival and the proliferation of the cell are assured.

### CITATION LIST

### PATENT DOCUMENT

Patent Document 1: US 2009/0298164 A1

### SUMMARY OF THE INVENTION

### PROBLEM TO BE SOLVED BY THE INVENTION

In replacing a culture medium in a culture vessel for adherent cells, the present inventors have newly found that the culture vessel is deformed to shrink when discharging the culture medium (natural flow-down through a tube utilizing height difference or pump suction) and to swell when injecting a fresh culture medium (injection by natural flow-down through a tube utilizing height difference or pump injection), and such deformation often causes a damage like a crack in the culture vessel. In the case where such a damage occurs, it is not possible to continue the culture, and all the various preparations and manipulations so far become useless. In addition, even if there is no damage, a cautious operation with highly careful attention is required to avoid the damage during replacement of the culture medium. These factors have an unignorable adverse influence on the efficiency of all the steps of a large scale culture of adherent cells.

This problem is caused by creation or negative pressure inside the culture vessel because of expansion of the gas phase capacity within the culture vessel as the medium is discharged from the culture vessel, and due to compression of the gas phase occurring inside the culture vessel because of reduction of the gas phase capacity within the culture vessel as a fresh culture medium is being injected. A membrane filter which is attached for exchanging gas phases between the inside and outside of the culture vessel is considered to have an insufficient gas permeability to cancel the changes in gas pressure caused in the culture vessel during medium exchange

In an attempt to solve the above problem, the present inventors adopted a depth filter through which gas can swiftly pass under pressure difference, instead of membrane filter, and found that deformation of culture vessel was avoided during medium exchange and thus there was no longer a concern about breakage.

However, it was observed in further studies that when cells were cultured in a culture vessel with an attached depth filter, instead of a membrane filter, the pH of the culture medium gradually lowered over time, with the concentration of CO₂ in the gas phase increasing in parallel, and it was found that CO₂ in the gas phase was extremely inhibited from dissipating outward through the depth filter. It was entirely unexpected that in spite that a depth filter, compared with a membrane filter, has such a large gas permeability that hardly creates pressure difference between the inside and the outside of a culture vessel during medium exchange as mentioned above, the depth filter shows substantially no outward permeability to carbon dioxide contained in the gas phase under a condition where there is no pressure difference between the inside and the outside.

Thus, an objective of the present invention is to provide a cell culture vessel possessing such benefits that it is protected from deformation during the process of loading/removing cells or medium exchange and that it prevents increase in carbon dioxide gas concentration and lowering of the pH of the culture medium over time during cell culture.

### MEANS FOR SOLVING PROBLEM

The present invention is completed through further studies based on the results mentioned above, and thus provides what follows.

1. A cell culture vessel comprising two different types of filters installed in parallel in a manner that each of the filters partitions between the internal gas phase and the external gas phase relative to the cell culture vessel.
2. The cell culture vessel according to 1 above, wherein at least one of the two types of filters is installed in the form of a filter unit, the filter unit defining a flow pass running therethrough and holding the filter in a manner that the filter transversely partitions the flow pass, wherein the filter unit is connected at one of the openings of the flow pass to a corresponding opening provided on a gas phase region of the body of the cell culture vessel.
3. The cell culture vessel according to 1 above, wherein each of the two types of filters is installed in the form of a filter unit, the filter unit defining a flow pass running therethrough and holding the filter in a manner that the filter transversely partitions the flow pass, wherein each of the filter unit is connected at one of the openings of the flow pass to a corresponding opening provided on a gas phase region of the body of the cell culture vessel.
4. The cell culture vessel according to 1 above, wherein the two types of filters are installed in a parallel-filter connector that defines an internal cavity having mutually communicating three openings, in a manner that each of the two types of filters transversely partition one of respective parallel flow paths running through two of the three openings, and wherein the parallel-filter connector is connected at the remaining openings to a corresponding opening provided on a gas phase region of the body of the cell culture vessel.
5. The cell culture vessel according to 4 above, wherein at least one of the two filters is installed in the form of a filter unit defining a flow pass running therethrough and holding the filter in a manner that the filter transversely partitions the flow pass, wherein the filter unit is connected at one of the openings of the flow pass to a corresponding opening provided on the parallel-filter connector.
6. The cell culture vessel according to 4 above, wherein each of the two filters is installed in the form of a filter unit, the filter unit defining a flow pass running therethrough and holding the filter in a manner that the filter transversely partitions the flow pass, wherein each of the filter units is connected at one of the openings of the flow pass to a corresponding opening provided on the parallel filter connector.
7. The cell culture vessel according to 2 or 3 above, wherein the filter unit and the body of the cell culture vessel are provided, at their respective openings to be connected to each other, with a part having a male screw thread and a corresponding part having a female screw thread, or with corresponding male and female tapered fitting surfaces.
8. The cell culture vessel according to 4 above, wherein the body of the cell culture vessel and the parallel-filter connector are provided, at their respective openings to be connected to each other, with a part having a male screw thread and a corresponding part having a female screw thread, or with corresponding male and female tapered fitting surfaces.
9. The cell culture vessel according to 5 or 6 above, wherein the filter unit, the body of the cell culture vessel, and the parallel-filter connector are provided, at their respective openings to be connected to one another, with a part having a male screw thread and a corresponding part having a female screw thread, or with corresponding male and female tapered fitting surfaces.
10. The cell culture vessel according to any one of 1 to 9 above, wherein the body of the vessel is provided further with an opening which is installed with a cap having a tube attached thereto.
11. The cell culture vessel according to any one of 4 to 6, 8, and 9 above, wherein the parallel filter connector is in the form of a generally branched tube having three ends each of which is provided with an opening.
12. The cell culture vessel according to any one of 1 to 11 above, wherein one of the two filters is a membrane filter and the other is a depth filter.
13. The cell culture vessel according to 12 above, wherein a thickness of the membrane filter is 100 to 200 µm, and a thickness of the depth filter is 210 to 800 µm.
14. The cell culture vessel according to 12 above, wherein a thickness of the membrane filter is 120 to 180 µm, and a thickness of the depth filter is 230 to 770 µm.
15. The cell culture vessel according to any one of 1 to 14 above, wherein a material of the cell culture vessel is a synthetic resin.
16. The cell culture vessel according to any one of 1 to 14 above, wherein a material of the cell culture vessel is selected from the group consisting of a polycarbonate resin, a polyester resin, a polystyrene resin, and an acrylic resin.
17. A parallel-filter connector to be connected to a body of a cell culture vessel, wherein the connector comprises an internal cavity having mutually communicating three openings in a manner that one of the three openings is configured to be connected to a corresponding opening provided in the body of the cell culture vessel, and the other two openings:
   (1) respectively hold one of two types of filters in a manner that each of the filter transversely partitions the internal cavity and outside;
   (2) are configured so that one of the opening is installed with one of two types of filters so as to transversely partition the internal cavity and outside, and the other opening is capable of being connected to a corresponding opening of a filter unit defining a flow pass running therethrough and holding the other of the two types of filters so as to transversely partition the flow pass; or
   (3) are configured so that each of the other two openings is capable of being connected to a corresponding opening of each of two filter units, the filter units defining a flow pass running therethrough and holding one of two types of filters in a manner that the filters transversely partition the flow pass.
18. The parallel-filter connector according to 17 above, wherein the parallel-filter connector is provided with, at the opening to be connected to an opening of a body of a cell culture vessel, a part having a male screw thread, a part having a female screw thread, a male tapered fitting surface, or female tapered fitting surface, so as to fit to the corresponding opening of a body of a cell culture vessel.
19. The parallel-filter connector according to 17 or 18 above, wherein the parallel-filter connector is in the form of a generally branched tube having the three opening at each of the terminuses thereof.
20. The parallel-filter connector according to any one of 17 to 19 above, wherein one of the two types of filters is a membrane filter, and the other is a depth filter.
21. The parallel-filter connector according to 20 above, wherein a thickness of the membrane filter is 100 to 200 µm, and a thickness of the depth filter is 210 to 800 µm.
22. The parallel-filter connector according to 21 above, wherein a thickness of the membrane filter is 120 to 180 µm, and a thickness of the depth filter is 230 to 770 µm.
23. The parallel-filter connector according to any one of 17 to 22 above, wherein the filter unit is connected to one or two of the three openings.
24. A kit of (1) or (2) below for combining the parallel-filter connector according to 23 above:
   (1) a kit including a parallel-filter connector installed with one of the two types of filters, and a filter unit installed with the other of the two types of filters;
   (2) a kit including a parallel-filter connector, a filter unit installed with one of the two types of filters, and a filter unit installed with the other of the two types of filters.

### EFFECT OF THE INVENTION

According to the present invention, it is possible to provide the cell culture vessel that is capable of preventing the deformation of the cell culture vessel such as swelling or shrinking, which occurs during the operation of replacing the culture medium in the cell culture vessel, and the consequent breaking of the cell culture vessel due to the deformation, and further is capable of maintaining pH of the culture medium in a suitable range during a culture period by preventing the accumulation of carbon dioxide in the cell culture vessel.

### BRIEF DESCRIPTION OF DRAWINGS

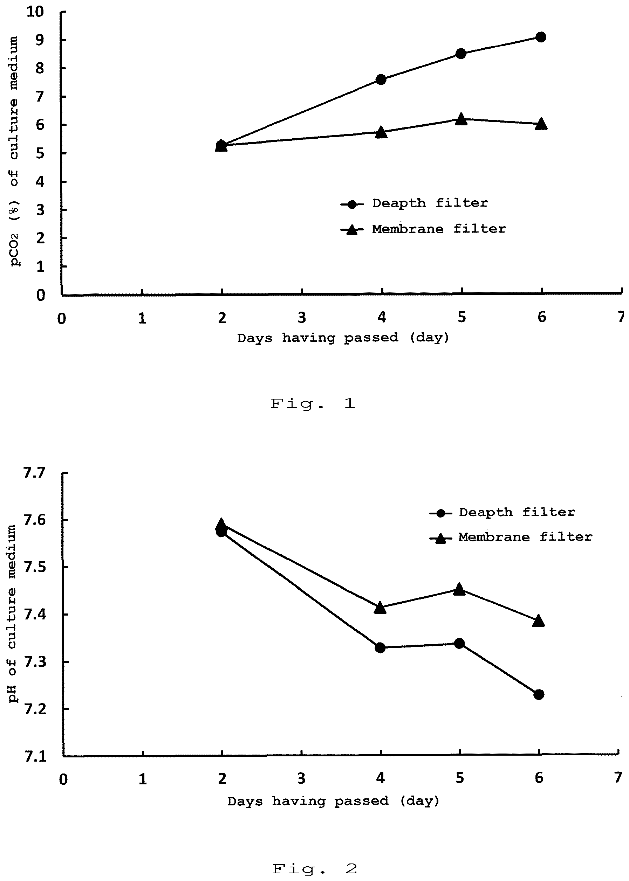

Fig. 1 is a graph illustrating a measurement of pCO₂ (%), as average values, of each culture medium during a culture period performed in a culture vessel which was obtained in Example 1 or Example 2 and installed with either a depth filter or a membrane filter. The vertical axis represents pCO₂ (%) of the culture medium, the horizontal axis represents days that have passed (days) from the start of culture, ● represents results (n = 2) obtained using the depth filter, and ▲ represents results (n = 4) obtained using the membrane filter;
Fig. 2 is a graph illustrating a measurement of pH, as average values, of a culture medium during a culture period performed in a culture vessel which was obtained in Example 1 or Example 2 and installed with either a depth filter or a membrane filter. The vertical axis represents pH of the culture medium, the horizontal axis represents days that have passed (days) from the start of the culture, ● represents results (n = 2) obtained using the depth filter, and ▲ represents an average (n = 4) of results obtained using the membrane filter;

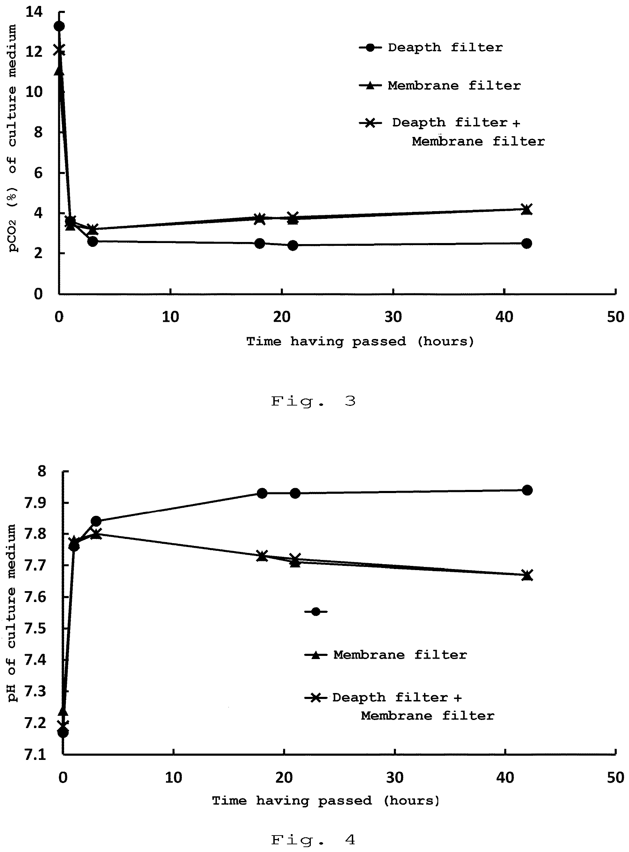

Fig. 3 is a graph illustrating a measurement of pCO₂ (%) of each culture medium in a test performed in a culture vessel installed only with a depth filter, only with a membrane filter, or both with depth and membrane filters via a parallel-filter connector obtained in Example 5. The vertical axis represents pCO₂ (%) of the culture medium, the horizontal axis represents time that has passed (hours) from the start of the test, ● represents results obtained using only the depth filter, ▲ represents results obtained using only the membrane filter, and × represents results obtained using the combination of both of the filters;
Fig. 4 is a graph illustrating a measurement of pH of each culture medium in an experiment performed using a culture vessel installed only with a depth filter, only with a membrane filter, or both with depth and membrane filters via a parallel-filter connector, obtained in Example 5. The vertical axis represents pH of the culture medium, the horizontal axis represents time that has passed (hours) from the start of the test, ● represents results obtained using only the depth filter, ▲ represents results obtained using only the membrane filter, and × represents results obtained using the combination of both of the filters;

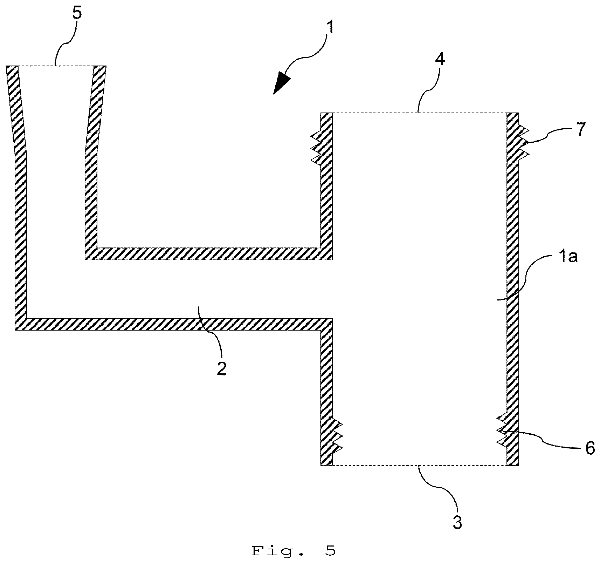

Fig. 5 illustrates a schematic sectional view of a parallel-filter connector in one embodiment of the invention;

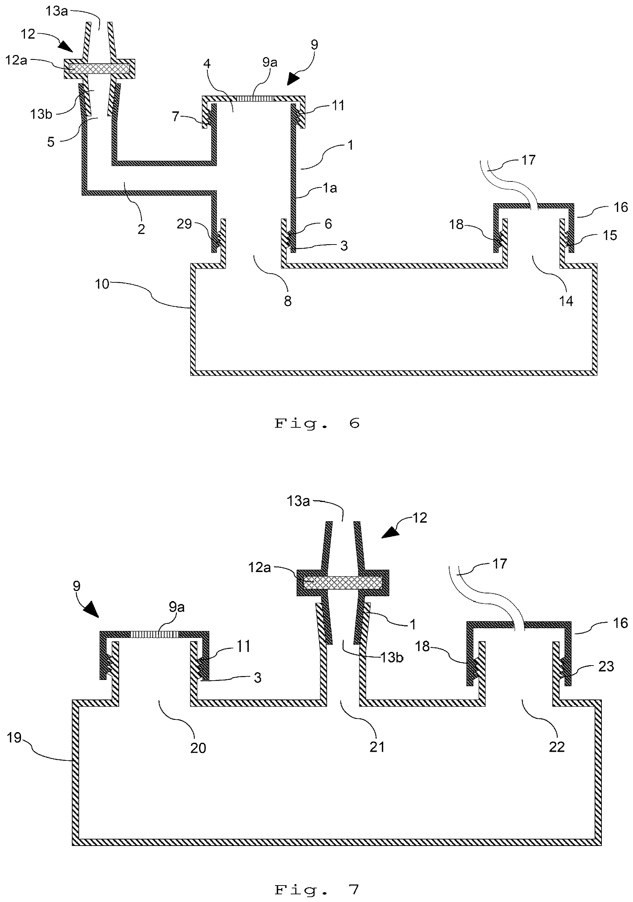

Fig. 6 illustrates a schematic sectional view of a body of a culture vessel in which the parallel-filter connector in Fig. 5 is attached to a first body opening and a cap with a tube is attached to a second body opening. A membrane filter unit is attached to a second opening of the parallel-filter connector and a depth filter unit is attached to a third opening;
Fig. 7 illustrates, as another embodiment of the invention, a schematic sectional view of a body of culture vessel having a first body opening to which a membrane filter unit is to be attached, a second body opening to which a depth filter is to be attached, and a third body opening to which a cap with a tube is to be attached. The membrane filter unit is connected to the first body opening, the depth filter unit is connected to the second body opening, and the cap with a tube is connected to the third body opening; and

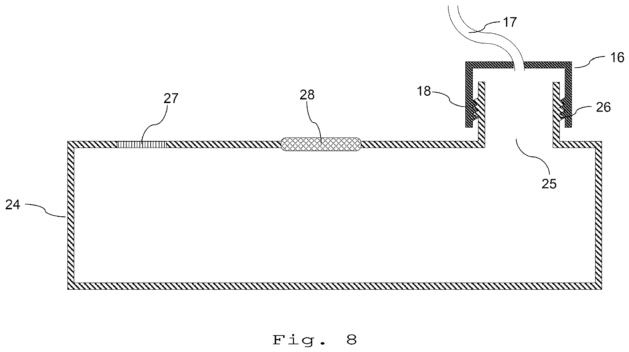

Fig. 8 illustrates, as one embodiment of the invention, a schematic sectional view of a culture vessel having a membrane filter and a depth filter and further having an opening to which a cap with a tube is attached.

### DESCRIPTION OF EMBODIMENTS

In this specification, the term "cell culture vessel" or "culture vessel" means a container in which an animal cell, a plant cell, or the like can be cultured while being retained in a state where the contamination with foreign substances such as microorganisms from the outside is prevented. The term "body of a cell culture vessel" or "culture vessel body" means a container in which a culture medium and cells are retained, and indicates a portion of "cell culture vessel" or "culture vessel" excluding a "filter".

One embodiment of a cell culture vessel included in the invention is a multi-layer culture vessel. The multi-layer culture vessel is for enabling a large scale culture in a shallow liquid depth while ensuring a wide adhesion area for cells, and it is a culture vessel having a multi-layer structure in which a plurality of trays are integrally fixed to be vertically stacked, wherein each tray has adhesion surfaces for cells and is formed into the shape of a shallow tray. As the surface of the support body (upper side) of each tray is accompanied by a space for accommodating a culture medium, as well as a gas phase contacting it, between the upper side of the tray and underside of a tray just above, and provides the adhesion surface to the cells, a wide cell adhesion area can be ensured corresponding to the number of trays. The multi-layer culture vessel, for example, has a multi-layer structure including 2 to 40 layers, 10 to 40 layers, 10 to 20 layers, 2 layers, 10 layers, 20 layers, 30 layers, and 40 layers of trays. In this specification, the cell adhesions surface in the culture vessel may be referred to as the culture surfaces of the culture vessel, or simply as culture surfaces.

Embodiments of the culture vessel of the invention are not limited to the multi-layer culture vessel described above, but also include a culture vessel for monolayer culture with a large area, for example, an adhesion area of 500 cm² or more, which may be preferably implemented. The adhesion area, for example, may be 500 to 2000 cm², 1000 to 2000 cm², or the like. Hereinafter, in this specification, unless otherwise specified, the term "culture vessel" includes both of a multi-layer culture vessel and a monolayer culture vessel. The material of the culture vessel is not particularly limited, but all or at least a part of it consists, for example, of a polycarbonate resin, a polyester resin, a polystyrene resin, or an acrylic resin.

In general, it is preferable that a culture vessel has a strength such that a breakage does not occur due to a distortion of the culture vessel which occurs when the inside of the culture vessel is decompressed or compressed, but according to the invention, it is possible to perform a manipulation for replacing the culture medium almost without decompressing or compressing the inside of the culture vessel. That is, according to the invention, it is possible to lower the required strength of the culture vessel, and thus, it is further possible to reduce the amount of the material required for producing a culture vessel, by thinning the outer wall of the culture vessel.

In one embodiment, a body of a culture vessel of the invention includes two or more openings, for example, two openings (first and second body openings), and three openings (first to third body openings). One of such openings functions as described in (1) below, and the remaining opening function as described in (2) below.
(1) An injection port for adding the culture medium and the cells to the culture vessel, and a discharge port for discharging the culture medium, the cell, and the like from the culture vessel.
(2) A vent for exchanging gas between the internal gas phase and the outer air without contaminating the inside of the culture vessel.

Among the openings of the body of the culture vessel, the one used for injecting and discharging the cells and the culture medium is provided with a lid such that air sealing is attained during the culture, and the lid is detached when injecting or discharging the cells or the culture medium. Instead of the lid, a cap having a flexible pass-through tube (hereinafter, referred to as a "cap with a tube") can also be attached. In this case, the tube of the cap with a tube is closed with a clip or the like during the cell culture. The injection or the discharge of the cells or the culture medium is conducted by connecting the terminal of the tube with a container accommodating the corresponding culture medium or a culture medium containing cells suspended in it, or an empty container, and detaching the aforementioned clip, via the tube (for example, by a peristaltic pump or the gravity). A condition where the inside of the container and the terminal of the tube are airtightly connected against the outer air enables aseptic injection and discharge of the cells or the culture medium to be easily performed. In the invention, the cap with a tube may also be integrally fixed to the opening of the container body used for the injection and the discharge of the culture medium and the cells.

In the other one or two openings in the body of the culture vessel, a suitable filter for enabling a gas to pass through but the contamination with foreign substances to be prevented is used to provide the function described in (2) above. In order to provide this function, both of a membrane filter and a depth filter are attached in the invention to configure a parallel flow pass between the gas phase in the culture vessel and the outer air. Here, "configuring the parallel flow pass" refers to not only configuring flow passes so that they are physically arranged side by side, but also configuring the flow passes so that each of two filters configure flow passes between the gas phase in the culture vessel and the external gas phase without passing through the other filter. Thus, insofar as each of the filters is in contact with the gas phase inside and outside the culture vessel, embodied examples are not limited thereto. Accordingly, the gas inside the body of the culture vessel of the invention can be exchanged with the outside through each of the filters independent from each other.

In the invention, the membrane filter is a film-shaped filter with circular holes ("pores") of approximately submicron order, the pores penetrating approximately vertically through the surface, having an approximately even inner diameter, and formed over the entire surface.

The pore diameter of the membrane filter is not particularly limited insofar as the membrane filter is capable of preventing the microorganisms or the like from passing through and allowing oxygen molecules and carbon dioxide molecules to pass through, but the pore diameter, for example, is 0.15 to 1.0 µm, 0.15 to 0.8 µm, 0.2 to 0.8 µm, 0.2 to 0.5 µm, 0.15 to 0.22 µm, 0.2 µm, or 0.22 µm. In addition, the thickness of the membrane filter is not particularly limited, but it is, for example, 120 to 180 µm or 100 to 200 µm.

The material of the membrane filter is not particularly limited, but a hydrophobic material such as polytetrafluoroethylene (PTFE), polyvinylidene fluoride resin (PVDF), or polyester, and a hydrophilic material such as nylon and cellulose can be preferably used. A hydrophilic material obtained by performing a hydrophilization treatment to a hydrophobic material can also be used as a material of the membrane filter. The hydrophobic material is particularly preferable as the material of the membrane filter. A membrane filter whose material has hydrophobicity is referred to as a hydrophobic membrane filter, and a membrane filter whose material has hydrophilicity is referred to as a hydrophilic membrane filter.

Along with metabolism during the cell culture, CO₂ is continuously discharged from the cell, not only in the case of using a multi-layer culture vessel, but also using a monolayer culture vessel such as a petri dish or a flask for cell culture. Therefore, in the case where a culture is performed in a fully air-tight culture vessel, CO₂ is accumulated in the culture vessel, and pH of the culture medium decreases along with an increase in the dissolved CO₂ concentration during the culture. By providing the membrane filter in the opening in the gas phase region of the culture vessel, the gas exchange between inside the culture vessel and outside the culture vessel occurs, and thus, the accumulation of CO₂ in the culture vessel is prevented, and a decrease in pH of the culture medium is also prevented.

In the case where the cells are mammal cells, in general, it is preferable that the carbon dioxide partial pressure (pCO₂ (%)) of a culture medium should be maintained at approximately 5%. In this specification, the "gas phase region" of the culture vessel is referred to as the region of the culture vessel in contact with the gas phase above the liquid surface of the culture medium during the cell culture. In addition, in this specification, the carbon dioxide partial pressure of the culture medium means the concentration of carbon dioxide in the gas phase in contact with the culture medium, which is obtained from a measurement value of the amount of dissolved carbon dioxide in the environment (the temperature, the atmospheric pressure, or the like) in which the culture medium is placed, by measuring the amount of dissolved carbon dioxide of the culture medium. Here, the exchange of carbon dioxide between the culture medium and the gas phase is in an equilibrium state.

The depth filter is a filter of which the internal filter material is a porous material, or a filter has a three-dimensional network structure inside. The material of the depth filter is not particularly limited, but for example, the material is a filter cloth, filter paper, a porous synthetic resin, a porous metal, asbestos, and the like. An example of the depth filter includes a filter produced by pressing together a glass fiber, a cellulose fiber, and the like. A hydrophobic glass fiber laminate having a three-layer structure of polyester, a glass fiber, and polyester can be preferably used as the depth filter.

A filter having the ventilation resistance per unit film area less than that of a membrane filter is used as the depth filter. The minimum diameter of the pore in the porous material of the depth filter or the minimum dimension of the gap in the three-dimensional network structure is not particularly limited insofar as the ventilation resistance per unit film area of the depth filter is less than that of the membrane filter, but a depth filter with a pore diameter identical to or greater than the pore diameter of the membrane filter, that membrane filter is attached directly in parallel to the culture vessel or simultaneously attached to the parallel-filter connector, can be preferably used. A depth filter of which the minimum diameter in the porous material thereof or the minimum dimension of the gap in the three-dimensional network structure is greater than the pore diameter of the membrane filter can be particularly preferably used. The minimum diameter of the pore of the depth filter or the minimum dimension of the gap, for example, is 0.5 to 1.5 µm, 0.8 to 1.2 µm, 0.8 to 1.0 µm, or 1.0 µm. For example, when the pore diameter of the membrane filter is 0.2 to 0.5 µm or 0.15 to 0.22 µm, the minimum diameter of the pore or the minimum dimension of the gap of the depth filter, for example, is 0.8 to 1.2 µm or 0.5 to 1.5 µm.

The thickness of the depth filter in a gas permeation direction is not particularly limited insofar as the ventilation resistance per unit film area of the depth filter is less than that of the membrane filter, and a depth filter with a thickness identical to or greater than the thickness of the membrane filter can be preferably used. A depth filter with a thickness greater than that of the membrane filter can be particularly preferably used. The thickness of the depth filter, for example, is 210 to 800 µm or 230 to 770 µm. For example, when the thickness of the membrane filter is 120 to 180 µm, the thickness of the depth filter is 210 to 800 µm.

In the depth filter, the pore diameter may decrease toward one side along the gas permeation direction. In this specification, the depth filter having such a structure will be referred to as a depth filter having directionality. In this case, in the depth filter, a filter surface on a side where the gas flows in is referred to as an inflow surface, and a filter surface on a side where the gas flows out is referred to as an outflow surface. In the invention, in a case where the depth filter having directionality is adopted, in general, the depth filter is disposed such that the inflow surface is on the outer air side, but the inflow surface may be on the gas phase side of the culture vessel. In addition, in the invention, a depth filter not having directionality can also be preferably used.

In another embodiment of the invention, as illustrated in Figs. 7 and 8, for example, in a case where the culture vessel body includes three openings, the membrane filter can be attached to one of two openings other than the opening used for injecting and discharging the culture medium or the cells, and the depth filter can be attached to the other, in a suitable manner. For example, the membrane filter and the depth filter can be directly attached by suitable means such as performing heat sealing or adhering of the circumferential edge with respect to the openings, or fixing by a ring-shaped member (Fig. 8). In addition, one or both of the two types of filters can also be airtightly connected to the culture vessel body in the form of a filter unit, which is a part installed inside with a filter (Fig. 7). The filter unit is a part that includes a pass-through flow pass and retains the filter disposed to partition the flow pass into a forward side and a backward side. The filter unit has an opening that can be airtightly connected to the corresponding opening of a connection partner such as a culture vessel body or the like, in at least one opening of the flow pass. That is, the openings to be a pair have the corresponding form for attaining airtight connection (for example, a male screw and a female screw, and a male-type tapered engagement surface or female-type tapered engagement surface), respectively.

The entire shape of the filter unit is not limited insofar as the shape is capable of corresponding to the shape of the opening of the connection partner, and for example, may be a suitable shape that can be attached to the opening provided on the top surface or the lateral surface of the culture vessel body, such as a cap shape capable of covering the opening of the connection partner, and a spinning-top shape in which a wide filter is disposed in the middle of the flow pass and the inner diameter is narrowed on both end sides of the flow pass. The portion of the filter unit other than the filter may contain polyvinyl chloride, or other suitable synthetic resins and/or metals. Here, even in a case where the culture vessel body has three openings, the parallel-filter connector can be used by being connected to one of the openings.

In addition, in a case where only two openings are provided in the culture vessel body, the membrane filter and the depth filter can be attached to the culture vessel body by using the opening (an opening for ventilation) that is not used for injecting or discharging the culture medium or the cells. In this case, the culture vessel of the invention includes the "parallel-filter connector" airtightly connected to the opening for ventilation, and the membrane filter and the depth filter are directly attached to the parallel-filter connector or are attached in parallel to the gas phase in the culture vessel in the form of each of the filter units. The "parallel-filter connector" is a part having an internal cavity with three openings, and one of the openings is airtightly connected to the opening with the corresponding shape provided in the culture vessel body. In addition, in the other two openings (communicating with the opening connected to the culture vessel body) of the parallel-filter connector, the membrane filter is independently attached to one and the depth filter is independently attached to the other by airtight connection, directly (heat sealing, adhering, or the like) or in the form of the filter unit. Accordingly, the opening of the parallel-filter connector, which is connected to the filter unit, has a shape suitable for the airtight connection with the opening of the filter unit (for example, a male screw and a female screw, and a male-type tapered engagement surface or female-type tapered engagement surface). Ventilation between the gas phase of the culture vessel and the outer air is attained through the two types of filters in parallel insofar as such characteristics are provided, and thus, the shape of the parallel-filter connector is not particularly limited. Accordingly, the entire shape of the parallel-filter connector, for example, can be any shape such as a branch tube shape including two openings other than the opening to be connected to the culture vessel body on the terminal, and a box shape or a dome shape including two openings other than the opening to be connected to the culture vessel.

As an example, in Fig. 6 and Fig. 7, a cap-shaped filter unit is illustrated as the filter unit including the membrane filter, and a spinning-top shaped filter unit is illustrated as the filter unit including the depth filter. Here, the entire shape and the shape of a connection merely correspond to the shape of the opening of the connection partner illustrated as an example in Fig. 6 and Fig. 7, and can be independently either a top shape or a cap shape insofar as the shape is capable of corresponding to the shape of the opening of the connection partner. In this specification, the filter unit retaining the membrane filter and the filter unit retaining the depth filter will be referred to as a "membrane filter unit" and a "depth filter unit", respectively.

Even in a case where the culture vessel body includes three or more openings, the parallel-filter connector can be used by being connected to one of the openings. In addition, a plurality of parallel-filter connectors can also be used by being connected to a plurality of openings of the culture vessel body.

In one embodiment of the invention, the parallel-filter connector is in the form of a generally branched tube, and includes a body and a branch extending from the lateral surface of the body. The body includes a first opening and a second opening, and the branch communicates with the body on one end and includes a third opening on the other end. The first opening is an opening for connecting with the opening provided in the culture vessel body (other than for the taking-in/out of the cells and the culture medium), the second opening is an opening for connecting with the first filter unit, and the third opening is an opening for connecting with the second filter unit.

An angle between the body and the origin of the branch may be any angle insofar as each of the first filter unit and the second filter unit can be sufficiently exhibited its function without physically or functionally interfering with each other, and for example, may be a right angle or any other angles. In addition, the inner diameters of two flow passes of the body and the branch may be the same or different from each other.

The entire shape can be formed such that the first opening of the parallel-filter connector is opened toward a horizontal direction or the lower side, and the second opening and the third opening thereof are opened to the horizontal direction or the upper side relative to the gas phase region of the culture vessel in accordance with the position (the lateral surface or the top surface) of the corresponding opening in the culture vessel body, when the first opening of the parallel-filter connector is connected to the culture vessel body.

The membrane filter unit is connected to one of the second and the third openings of the parallel-filter connector, and the depth filter unit is connected to the other. It is preferable that the second and the first openings of the parallel-filter connector are provided in reverse directions on both ends of the body, respectively. In order for ventilation between inside and outside the culture vessel in a still standing state, it is preferable that the membrane filter unit is attached to the second opening, but the invention is not limited thereto, and the depth filter unit may be attached to the second opening.

The first to third openings of the parallel-filter connector, for example, include a female screw, a male screw, a male-type tapered engagement surface, or female-type tapered engagement surface, corresponding to the shape of the opening of the filter unit, such that the first to third openings can be connected to the culture vessel or the filter unit. Here, the shape of the opening for mutual connection may be one of any other shapes insofar as mutual airtight connection is attained. For example, the opening of the parallel-filter connector and the opening on the terminal of the filter unit may be connected by pushing a tube-shaped connector made of a synthetic resin with flexibility and elasticity such as soft polyvinyl chloride or through a connector containing other synthetic resins or metals. The same applies to the connection between the parallel-filter connector and the culture vessel body.

In a case where the parallel-filter connector and the membrane filter unit and/or the depth filter unit are connected through such a connector, the shape of the connector can be adjusted to have a linear shape, a curved shape, an L shape, and any other suitable shapes such that each of the filter units and the culture vessel body or the filter units do not physically interfere with each other when the parallel-filter connector is connected to the culture vessel body.

The connection between the parallel-filter connector and each of the filter units via the connector may be performed by forming male and female to be screwed together at a junction between the connector and the parallel-filter connector and between the connector and each of the filter units, or by welding or any other suitable fixation means.

It is assumed that the parallel-filter connector that can be connected to the culture vessel body is used in an aseptic environment. Accordingly, it is necessary that the parallel-filter connector is subjected to a sterilization treatment before use. In a case where the parallel-filter connector includes a plurality of parts containing different materials, the materials of all of the parts are required to have durability with respect to at least any one of gamma sterilization, dry air sterilization, high-pressure steam sterilization, methylene oxide gas (EOG) sterilization, low-temperature gas plasma sterilization, or the like. The material may be a synthetic resin or rubber such as a heat-resistant synthetic resin, a metal such as stainless steel, glass, ceramics, and the like insofar as deformation, fusion, expansion, contraction, alteration, deterioration, rupture, decomposition, collapse, corrosion, ignition, explosion, and the like do not occur before and after the sterilization treatment. Here, the material is required to ensure the airtightness of each connection such that an aseptic state is entirely maintained when connecting parallel-filter connector thereto the filter unit is connected to the culture vessel.

The parallel-filter connector can be supplied as individually packaged. In a case where the parallel-filter connector is provided with the internal cavity including three openings communicating with each other, in which one of the three openings is connected to the corresponding opening provided in the body of the cell culture vessel, and in the other two openings, one opening includes one of the two types of filters to partition the internal cavity from the outside, and the other opening is connected to the corresponding opening of the filter unit that includes the pass-through flow pass and retains the other of the two types of filters to divide the flow pass into a forward side and a backward side, the parallel-filter connector can be supplied as packaged together with the filter unit, that it, as a kit. In a case where the parallel-filter connector includes the membrane filter, the filter unit included in the kit is installed with the depth filter. On the other hand, in a case where the parallel-filter connector includes the depth filter, the filter unit included in the kit is installed with the membrane filter. In such a kit, a lid for partitioning the inside and the outside of the parallel-filter connector may be attached to the opening of the parallel-filter connector not attached one of the filter units, that is, the opening for attaching the other filter unit. The lid is detached when attaching the other filter unit.

In a case where the parallel-filter connector is provided with the internal cavity including three openings communicating with each other, in which one of the three openings is connected to the corresponding opening provided in the body of the cell culture vessel, and the other two openings are connected to the corresponding openings of two filter units each of which includes a pass-through flow pass and retain the one or the other of the two types of filters to partition the pass flow into a forward side and a backward side, the parallel-filter connector can be supplied by being packaged together with the two filter units, that is, as a kit. In the case of such a kit, a lid for partitioning the inside and the outside of the parallel-filter connector may be attached to the opening of the parallel-filter connector for attaching the filter unit. The lid is detached when attaching the filter unit.

In the invention, in substation for the depth filter, the membrane filter with a sufficiently large effective filtration area can also be used. Here, the membrane filter with a sufficiently large effective filtration area is referred to as a membrane filter which generates in the membrane identical to or less ventilation resistance than that generated in the depth filter when the culture medium is discharged from the cell culture vessel.

That is, in one embodiment of the invention, the membrane filter unit is connected to one of the second and the third opening of the parallel-filter connector, and the membrane filter unit having the membrane filter with a sufficiently large effective filtration area is connected to the other. Preferred examples of the membrane filter with a sufficiently large effective filtration area include a LABODISC (Registered Trademark) disposable membrane filter unit (manufactured by ADVANTEC TOYO KAISHA, LTD., a pore diameter of 0.2 µm, an effective filtration area of 19.6 cm²).

### EXAMPLES

Hereinafter, the invention will be described in more detain with reference to examples, but it is not intended that the invention is limited to the examples.

### [Example 1] Cell Culture Using Culture vessel with Depth Filter

In CellSTACK (Registered Trademark) (10 chambers, manufactured by Corning Incorporated), which is a multi-layer culture vessel including two openings, a screw cap with a tube was attached to one of openings, and a cap installed with a depth filter (manufactured by Pall Corporation, a air vent of 37 mm, a nominal pore diameter of 1 µm, an effective filtration area of 7.5 cm²) was attached to the other opening (a culture vessel with a depth filter). The bacterial air vent of 37 mm is a depth filter having a hydrophobic glass fiber laminate film with a three-layer structure of polyester/glass fiber/polyester, and a flow rate when allowing the air to pass through the filter at a differential pressure of 40 kPa is 40 L/minute. CellSTACK (10 chambers) is a culture vessel including an adhesion surface of 6360 cm² for enabling adhesion and proliferation of cells. A suspension of a dental pulp-derived cell suspended in a culture medium was added such that the number of cells per unit area was approximately 1.6 × 10⁴ pieces/cm² relative to the adhesion surface. The amount of culture medium was to be 1500 mL. As the culture medium, a culture medium prepared by adding fetal bovine serum (FBS, manufactured by GE HealthCare) to DMEM Low Glucose (manufactured by GE HealthCare, a glucose concentration of 5.56 mM) to the final concentration of 10% was used. The culture vessel was left to still in a CO₂ incubator in which a CO₂ concentration was set to 5%, and culture was started at 37°C in a wet condition. The opening of the tube of the cap with a tube was airtightly closed with a clip during a culture period. A test was performed by using two culture vessels.

As the multi-layer culture vessel, CellSTACK (manufactured by Corning Incorporated) was used, but the same test can be performed by using commercially available other multi-layer culture vessels such as Cell Factory TM (manufactured by Thermo Fisher Scientific Inc.) and BioFactory TM (manufactured by Wuxi NEST Biotechnology Co., Ltd.).

### [Example 2] Cell Culture Using Culture vessel with Membrane Filter

In CellSTACK (10 chambers, manufactured by Corning Incorporated) that is a multi-layer culture vessel including two openings, a cap with a tube was attached to one opening, and a membrane filter (manufactured by Corning Incorporated, a 33 mm-vent cap for CellSTACK, a pore diameter of 0.2 µm) was attached to the other opening (a culture vessel with a membrane filter). The 33 mm-vent cap for CellSTACK is a membrane filter of which the material is high-density polyethylene. The same suspension of a dental pulp-derived cell suspended in a culture medium as used in Example 1 was added such that the number of cells per unit area was approximately 1.6 × 10⁴ pieces/cm² relative to an adhesion surface of CellSTACK (10 chambers). The amount of culture medium was to be 1500 mL. As the culture medium, a culture medium prepared by adding fetal bovine serum (FBS) (manufactured by GE HealthCare) to DMEM Low Glucose (manufactured by GE HealthCare, a glucose concentration of 5.56 mM) to the final concentration of 10% was used. The culture vessel was left to still in a CO₂ incubator in which a CO₂ concentration was set to 5%, and culture was started at 37°C in a wet condition. The opening of the tube of the cap with a tube was airtightly closed with a clip during a culture period. A test was performed by using four culture vessels.

### [Example 3] Measurement of Carbon Dioxide Partial Pressure and pH of Culture Medium

In the cell culture of Examples 1 and 2, the starting date of the culture was set to Day0, and 2, 4, 5 and 6 days after the start of the culture was respectively set to Day2, Day4, Day5. 5 mL of the culture medium was sampled by using a 30 mL-syringe (with a lock, manufactured by Terumo Corporation) aseptically joined to the tube of the cap with a tube at each of Day2, and Day4 to Day6, and the carbon dioxide partial pressure (pCO₂ (%)) and pH of the culture medium were measured by using a pH/PCO₂/PO₂ analyzer BioProfile pHOx (Registered Trademark) (manufactured by Nova Biomedical Corporation), and the change in such values over time was observed. The measurement was implemented following the instruction handbook of BioProfile pHOx. As the measurement values of the carbon dioxide partial pressure (pCO₂ (%)) and pH were greatly changed depending on the temperature of a measurement sample, the measurement should be performed by keeping the measurement sample at a constant temperature with a heat source in the pH/PCO₂/PO₂ analyzer BioProfile pHOx, and thus the setting temperature was set to 37°C. The measurement was performed for all of the culture vessels used in the test, and the average of the measurement values was calculated. The carbon dioxide partial pressure is the concentration of carbon dioxide in the gas phase in contact with the culture medium, which is obtained from the measurement value of the amount of dissolved carbon dioxide in the environment (the temperature, the atmospheric pressure, or the like) in which the culture medium is placed by measuring the amount of dissolved carbon dioxide of the culture medium.

In the cell culture (using the culture vessel with a depth filter) of Example 1 and the cell culture (using the culture vessel with a membrane filter) of Example 2, the measurement of pCO₂ (%) of the culture medium is illustrated in Fig. 1, and the measurement of pH is illustrated in Fig. 2. Up to Day2, pCO₂ (%) of both of the culture medium cultured by using the culture vessel with a depth filter and with a membrane filter was approximately same, that is, approximately 5%. However, in the case of performing the culture by using the culture vessel with a depth filter, pCO₂ (%) of the culture medium continuously increased, and reached approximately 9% at Day6. On the other hand, in the case of performing the culture by using the culture vessel with a membrane filter, pCO₂ (%) of the culture medium slightly increased, but was still approximately 6% at Day6.

In addition, up to Day2, pH of both of the culture medium cultured by using the culture vessel with a depth filter and with a membrane filter was approximately 7.6, which was approximately the same. However, after Day4, pH of the culture medium cultured by using the culture vessel with a depth filter was lower than pH of the culture medium cultured by using the culture vessel with a membrane filter, and at Day6, pH of the former was approximately 7.23, and pH of the latter was approximately 7.40. A correlation between the values of pCO₂ (%) and pH of the culture medium can be observed, that is, when pH decreases, pCO₂ (%) of the culture medium increases. This is because that a part of CO₂ forms carbon dioxide ion or the like in an aqueous solution.

The results described above indicate that in a case where the cell culture is performed by using the culture vessel with a depth filter, an increase in pCO₂ (%) of the culture medium is not capable of being suppressed, and thus, pH greatly decreases. Since the CO₂ concentration in the CO₂ incubator is 5%, when the culture is started, the CO₂ concentration of the gas phase in the culture vessel has to be also 5%, and thus, pCO₂ (%) of the culture medium is approximately 5%. During the cell culture period, CO₂ is continuously produced by the respiration of the cells and is discharged into the culture medium, and thus, pCOz (%) of the culture medium tends to increase, compared to the time of onset of the culture. In order to suppress an increase in pCOz (%) of the culture medium and to maintain pCO₂ (%) of the culture medium at approximately 5 to 6%, it is necessary to maintain the CO₂ concentration of the gas phase in the culture vessel at approximately 5%. While oxygen contained in the gas phase of the culture vessel is consumed by the cells, CO₂ produced by the cells is discharged from the liquid surface of the culture medium, and thus, in the case where gas exchange (static gas exchange) is not sufficiently performed between the gas phase in the culture vessel and the gas phase in the CO₂ incubator, the CO₂ concentration of the gas phase increases, resulting that an increase in pCO₂ (%) of the culture medium, which is in an equilibrium relationship with an increase in the CO₂ concentration, can't be prevented.

As illustrated in Fig. 1, in the case of performing the culture by using the culture vessel attached only with a membrane filter, pCO₂ (%) in the culture medium during the culture period is maintained at 5 to 6%. It is considered that this is because the gas exchange is sufficiently performed between the gas phase in the COz incubator and in the culture vessel through the membrane filter, resulting that the COz concentration in the culture vessel is maintained at 5 to 6%. On the other hand, unexpectedly, in the case of performing the culture by using the culture vessel attached only with the depth filter, pCO₂ (%) in the culture medium during the culture period increased to approximately 9%. It is considered that this is because the gas exchange between the gas phase in the COz incubator and in the culture vessel, and at least the exchange of carbon dioxide gas, is not sufficiently performed by the depth filter.

From the aspect of maintaining pCO₂ (%) of the culture medium, the results described above indicate the depth filter is not suitable for enabling sufficient gas exchange between the inside of the culture vessel and the space in the CO₂ incubator, thus the membrane filter is to be used.

### [Example 4] Collection of Culture Medium

In the cell culture of Examples 1 and 2, in 6 days from the starting date of the culture, the culture vessel was taken out from the inside of the CO₂ incubator, and the clip of the tube was detached to connect the terminal of the tube to a culture medium collection container.

By obliquely lifting up the culture vessel with a depth filter of Example 1, the culture medium was discharged and collected to the culture medium collection container by the gravity flow. The culture medium was capable of being discharged from the culture vessel at a speed of approximately 290 mL/minute (calculated by dividing the total amount of the culture media by time spent for discharging the solution). In contrast, in the culture vessel with a membrane filter of Example 2, when the culture medium was similarly discharged from the culture vessel, the culture vessel cracked. Therefore, it was not substantially possible to discharge the culture medium by the gravity flow.

In a case where the culture medium is discharged from the culture vessel, the volume of the gas phase in the culture vessel increases by the amount of liquid discharged. Accordingly, in a case where the outer air with the same volume does not flow in the culture vessel, the inside of the culture vessel becomes in a decompressed state, and the pressure from the outside is exerted on the outer wall of the culture vessel. It is considered that the crack occurs in the culture vessel with a membrane filter because the inflow of the outer air does not occur at the speed for discharging culture medium by the gravity flow through the membrane filter, and thus, the pressure inside the culture vessel becomes excessively negative, a large load due to the external atmospheric pressure is applied to the culture vessel, and the culture vessel is distorted over the limit. In contrast, it is considered that in the culture vessel with a depth filter, the outer air flows into the culture vessel at a speed sufficient to prevent the decompression in the culture vessel when the culture medium is discharged by the gravity flow.

The results described above indicate that as a filter to be attached to the culture vessel, the membrane filter is not suitable, and the depth filter is preferable, from the aspect of aseptically and smoothly collecting the culture medium and of preventing a damage in the culture vessel.

In total, from the results of Examples 1 to 4, in the case of performing the cell culture by using only one of the depth filter and the membrane filter as the filter between the gas phase inside and outside the culture vessel, it was found that there were previously unknown shortcomings in use of the filters such as disabling compatibility between two requirements, that is, for enabling pCO₂ (%) and pH of the culture medium to be maintained during the culture period in a certain range, and the culture medium to be smoothly discharged from the culture vessel.

### [Example 5] Evaluation of Culture vessel attached with Both of Depth Filter and Membrane Filter

On the basis of the results described above, a culture vessel was prepared in which a parallel-filter connector installed with both of a depth filter or a membrane filter was connected to the opening of the culture vessel body to which the depth filter or the membrane filter was attached above, and conducted was the evaluation whether the shortcomings of the membrane filter and the depth filter could be complemented each other. The culture vessel is described in detail in Example 6. The evaluation of the culture vessel installed with both of the depth filter and the membrane filter was performed by using a multi-layer culture vessel to which the filters were attached and to which only the culture medium was added without using cells, unlike examples 1 and 2.

1500 mL of a culture medium in which fetal bovine serum (FBS) (manufactured by GE HealthCare) was added to DMEM Low Glucose (manufactured by GE HealthCare, a glucose concentration of 5.56 mM) to the final concentration of 10% was added to each of three CellSTACKs (10 chambers, manufactured by Corning Incorporated) that are a multi-layer culture vessel having two openings. Next, a cap with a tube was attached to one opening of each of CellSTACKs to be connected to another container. Any one of (i) a depth filter (a cap with a bacterial air vent of 37 mm, manufactured by Pall Corporation, a nominal pore diameter of 1 µm), (ii) a membrane filter (a 33 mm-vent cap for CellSTACK, manufactured by Corning Incorporated, a pore diameter of 0.2 µm), and (iii) a parallel-filter connector to which both of the depth filter and the membrane filter were connected, was attached to the other opening.

Here, the culture vessel to which the depth filter was attached, the culture vessel to which the membrane filter was attached, and the culture vessel to which the parallel-filter connector to which both of the depth filter and the membrane filter were attached was connected will be referred to as a first culture vessel, a second culture vessel, and a third culture vessel, respectively.

The CellSTACKs were stored in a wet CO₂ incubator in which the CO₂ concentration was set to 5%. At 0 hour, at 1 hour, at 3 hours, at 18 hours, at 21 hours, and at 42 hours from the storage, 5 mL of the culture medium was sampled by using a 30 mL-syringe (with a lock, manufactured by Terumo Corporation) aseptically joined to the tube of the cap with a tube, and the carbon dioxide partial pressure (pCO₂) and pH of the culture medium were measured by using a pH/PCO₂/PO₂ analyzer BioProfile pHOx (manufactured by Nova Biomedical Corporation). The measurement was generally implemented following the instruction handbook of BioProfile pHOx. As the measurement values of the carbon dioxide partial pressure (pCO₂ (%)) and pH were greatly changed depending on the temperature of a measurement sample, the measurement should be performed by keeping the measurement sample at a constant temperature with a heat source in the pH/PCO₂/PO₂ analyzer BioProfile pHOx, and thus the setting temperature was set to 37°C.

The measurement of pCO₂ (%) of the culture medium added into the first to third culture vessels is illustrated in Fig. 3, and the measurement of pH is illustrated in Fig. 4. In all of the culture medium, within 2 hours, pCO₂ (%) decreased, and pH increased. It is considered that this is because CO₂ contained in the culture medium was deaerated after the culture vessel had been left to stand in the incubator. Subsequently, in the culture medium in the second and third culture vessels having the membrane filter, pCO₂ (%) increased, and pH decreased, and it is considered that this is because CO₂ was supplied from the outer air (the gas phase in the incubator) through the membrane filter. On the other hand, in the culture medium in the first culture vessel to which only the depth filter was attached, it was found that pCO₂ (%) rarely increased, and pH continuously increased within 18 hours from the start of the measurement, and then, decreased. It is considered that this is because gas exchange by natural air flow was not performed between the gas phase in the culture vessel and the outer air through the depth filter, and the supply of CO₂ from the outer air is little, and thus, the effect of deaerating CO₂ contained in the culture medium continues.

Next, CellSTACK was taken out from the incubator, the culture medium collection container was connected to the cap with a tube attached to CellSTACK, and the culture medium was discharged by the gravity flow. The culture medium was capable of being discharged at a speed of approximately 290 mL/minute (calculated by dividing the total amount of the culture media by time spent for discharging the solution) in the first culture vessel, and the culture medium was capable of being discharged at a speed of approximately 280 mL/minute in the third culture vessel, whereas in the second culture vessel to which only the membrane filter was attached, the inside of the culture vessel became in a decompressed state when the culture medium was discharged, resulting in occurrence of distortion and damage in the culture vessel, which could be visually checked, and thus, the culture medium was not capable of being discharged in a usual way.

The results described above indicate that it is possible to make two requirements, that is, for enabling pCOz (%) and pH of the culture medium to be maintained during the culture period in a certain range, and the culture medium to be smoothly discharged from the culture vessel compatible with each other by attaching the parallel-filter connector to which both of the depth filter and the membrane filter were attached to one of the openings of the culture vessel body.

### [Example 6] One Embodiment of Parallel-filter Connector

Fig. 5 illustrates a schematic sectional view of the parallel-filter connector used in Example 5. A parallel-filter connector (1) is in the shape of a generally branch tube, and has a first opening (3), a second opening (4), and a third opening (5). The parallel-filter connector (1) has a body (1a), and a branch (2) provided in the middle of the lateral wall of the body. The branch (2) communicates with the inside of the body (1a). The body (1a) has the first opening (3) for connecting the body to the opening of the culture vessel body, and the second opening (4) for attaching the membrane filter. In the first opening (3), a female screw (6) for airtightly screwing the parallel-filter connector to the opening of the culture vessel body is provided. In the second opening (4), a male screw (7) for airtightly screwing the membrane filter unit is provided. The third opening (5) in the branch (2) includes a female-type tapered engagement surface to be airtightly engaged with the depth filter unit (having a male-type tapered engagement surface for connection).

### [Example 7] Parallel-filter Connector

Fig. 6 illustrates a schematic sectional view of the parallel-filter connector (1) in the state of being attached to a culture vessel body (10) used in Example 5. The female screw (6) provided in the first opening (3) is screwed to a male screw (29) provided in a first body opening (8) of the culture vessel body (10), and thus, the parallel-filter connector (1) is airtightly connected to the culture vessel body (10). In the drawing, the structure of the culture vessel body (10) as an internal multi-layer culture vessel is omitted.

A membrane filter unit (9) is airtightly connected to the second opening (4) of the parallel-filter connector (1). In this example, the membrane filter unit (9) is views as a flat cylindrical lid, and a membrane filter (9a) is attached to an opening provided on the top surface thereof, and thus, the outer air and the gas phase in the parallel-filter connector (1), and further, the gas phase in the culture vessel body (10) can be circulated. The airtight connection between the parallel-filter connector (1) and the membrane filter unit (9) is attained by screwing a female screw (11) provided in an opening underside of the membrane filter unit (9) and the male screw (7) provided in the second opening (4) of the parallel-filter connector (1) together.

A depth filter unit (12) is airtightly connected to the third opening (5) of the parallel-filter connector (1). In this example, the depth filter unit (12) is in spinning-top shape, has a depth filter (12a) in a flow pass, has one opening (13a and 13b) on each of the upper side and the lower side, and an engagement surface thereof with an outer shape tapered toward the opening (13b) on the lower terminal is airtightly engaged with complementary engagement surface tapered toward the lower side inside the third opening (5) of the parallel-filter connector (1). The outer air and the gas phase in the parallel-filter connector (1), and further, the gas phase in the culture vessel body (10) can be circulated through the depth filter (12a).

A cap (16) with a tube is airtightly connected to a second body opening (14) of the culture vessel. A tube (17) of the cap (16) with a tube extends through the top surface of the cap, and allows the inside of the culture vessel body (10) to communicate with the outside. The connection between the second body opening (14) of the culture vessel and the cap (16) with a tube is attained by screwing together a male screw (15) provided in the second body opening (14) and a female screw (18) provided in the cap (16) with a tube.

An opening on the terminal outside the tube (17) is closed with a clip during the cell culture. When the culture medium is collected from the culture vessel, the clip is detached, and the culture vessel is inclined by 90 degrees to 180 degrees relative to a horizontal surface, and thus, the culture medium can be discharged from the opening of the tube by the gravity flow. Alternatively, the culture medium can be discharged by connecting the tube (17) to a peristaltic pump.

### [Example 8]

Fig. 7 illustrates another example of the culture vessel. A culture vessel body (19) has a first body opening (20), a second body opening (21), and a third body opening (22), the membrane filter unit (9) is attached to the first opening (20), and the depth filter unit (12) is attached to the second opening (21). In the drawing, as an example, it is illustrated that the first opening (20) has a male screw for connecting the membrane filter unit (9), and the second opening (21) has a female-type tapered engagement surface for connecting the depth filter unit (12). Here, the form of the connection can be selected in accordance with various forms of the connection of each filter unit.

The culture vessel (19) illustrated in Fig. 7 has the third body opening (22), which is an opening for injecting and discharging the cells and the culture medium, similar with the second body opening (14) illustrated in Fig. 6, and further similarly, the cap (16) with a tube (17) is airtightly connected to the third body opening. Here, the form of the connection of the third opening (22) can also be selected in accordance with various forms of the connection of the cap with a tube.

### [Example 9]

Fig. 8 illustrates another example of the invention. In this example, the membrane filter and the depth filter are integrally attached to a culture vessel (24) as a part thereof. In the culture vessel (24), both of a membrane filter (27) and a depth filter (28) are attached to the culture vessel body (24) in a parallel relationship such that gas exchange between the internal gas phase and the outer air can be achieved. An opening (25) of the culture vessel (24) is an opening for injecting and discharging the cells and the culture medium, similar with the second opening (14) illustrated in Fig. 6, has a male screw (26), and further similarly, the cap (16) with a tube (17) is airtightly connected to the opening. The form of the connection of the opening (25) can also be selected in accordance with various forms of the connection of the cap with a tube.

### INDUSTRIAL APPLICABILITY

According to the invention, a cell culture vessel can be provided which has advantages that deformation such as the swelling or the shrinking of the culture vessel does not occur during the operation of exchanging the culture medium, and pH of the culture medium maintains in a suitable range during the culture period by preventing the accumulation of carbon dioxide in the culture vessel. In addition, the parallel-filter connector of the present inventions can be used as means for upgrading the culture vessel having only one opening for gas exchange to a culture vessel with the same advantages as described above.

### EXPLANATIONS OF LETTERS OR NUMERALS

- 1: PARALLEL-FILTER CONNECTOR
- 1a: BODY
- 2: BRANCH
- 3: FIRST OPENING
- 4: SECOND OPENING
- 5: THIRD OPENING
- 6: FEMALE SCREW
- 7: MALE SCREW
- 8: FIRST BODY OPENING
- 9: MEMBRANE FILTER UNIT
- 9a: MEMBRANE FILTER
- 10: CULTURE VESSEL BODY
- 11: FEMALE SCREW
- 12: DEPTH FILTER UNIT
- 12a: DEPTH FILTER
- 13a: OPENING
- 13b: OPENING
- 14: SECOND BODY OPENING
- 15: MALE SCREW
- 16: CAP WITH TUBE
- 17: TUBE
- 18: FEMALE SCREW
- 19: CULTURE VESSEL BODY
- 20: FIRST BODY OPENING
- 21: SECOND BODY OPENING
- 22: THIRD BODY OPENING
- 23: LATCH SCREW
- 24: CULTURE VESSEL
- 25: OPENING
- 26: MALE SCREW
- 27: MEMBRANE FILTER
- 28: DEPTH FILTER
- 29: MALE SCREW

## Claims

1. A cell culture vessel comprising two types of filters installed in parallel in a manner that each of the filters partitions between the internal gas phase and the external gas phase relative to the cell culture vessel.

2. The cell culture vessel according to claim 1, wherein at least one of the two types of filters is installed in the form of a filter unit, the filter unit defining a flow pass running therethrough and holding the filter in a manner that the filter transversely partitions the flow pass, wherein the filter unit is connected at one of the openings of the flow pass to a corresponding opening provided on a gas phase region of the body of the cell culture vessel.

3. The cell culture vessel according to claim 1, wherein each of the two types of filters is installed in the form of a filter unit, the filter unit defining a flow pass running therethrough and holding the filter in a manner that the filter transversely partitions the flow pass, wherein each of the filter unit is connected at one of the openings of the flow pass to a corresponding opening provided on a gas phase region of the body of the cell culture vessel.

4. The cell culture vessel according to claim 1, wherein the two types of filters are installed in a parallel-filter connector that defines an internal cavity having mutually communicating three openings, in a manner that each of the two types of filters transversely partitions one of respective parallel flow paths running through two of the three openings, and wherein the parallel-filter connector is connected at the remaining openings to a corresponding opening provided on a gas phase region of the body of the cell culture vessel.

5. The cell culture vessel according to claim 4, wherein at least one of the two filters is installed in the form of a filter unit defining a flow pass running therethrough and holding the filter in a manner that the filter transversely partitions the flow pass, wherein the filter unit is connected at one of the openings of the flow pass to a corresponding opening provided on the parallel-filter connector.

6. The cell culture vessel according to claim 4, wherein each of the two filters is installed in the form of a filter unit, the filter unit defining a flow pass running therethrough and holding the filter in a manner that the filter transversely partitions the flow pass, wherein each of the filter units is connected at one of the openings of the flow pass to a corresponding opening provided on the parallel filter connector.

7. The cell culture vessel according to claim 2 or 3, wherein the filter unit and the body of the cell culture vessel are provided, at their respective openings to be connected to each other, with a part having a male screw thread and a corresponding part having a female screw thread, or with corresponding male and female tapered fitting surfaces.

8. The cell culture vessel according to claim 4, wherein the body of the cell culture vessel and the parallel-filter connector are provided, at their respective openings to be connected to each other, with a part having a male screw thread and a corresponding part having a female screw thread, or with corresponding male and female tapered fitting surfaces.

9. The cell culture vessel according to claims 5 or 6, wherein the filter unit, the body of the cell culture vessel, and the parallel-filter connector are provided, at their respective openings to be connected to one another, with a part having a male screw thread and a corresponding part having a female screw thread, or with corresponding male and female tapered fitting surfaces.

10. The cell culture vessel according to any one of claims 1 to 9, wherein the body of the vessel is provided further with an opening which is installed with a cap having a tube attached thereto.

11. The cell culture vessel according to any one of claims 4 to 6, 8, and 9, wherein the parallel filter connector is in the form of a generally branched tube having three ends each of which is provided with an opening.

12. The cell culture vessel according to any one of claims 1 to 11, wherein one of the two filters is a membrane filter and the other is a depth filter.

13. The cell culture vessel according to claim 12, wherein a thickness of the membrane filter is 100 to 200 µm, and a thickness of the depth filter is 210 to 800 µm.

14. The cell culture vessel according to claim 12, wherein a thickness of the membrane filter is 120 to 180 µm, and a thickness of the depth filter is 230 to 770 µm.

15. The cell culture vessel according to any one of claims 1 to 14, wherein a material of the cell culture vessel is a synthetic resin.

16. The cell culture vessel according to any one of claims 1 to 14, wherein a material of the cell culture vessel is selected from the group consisting of a polycarbonate resin, a polyester resin, a polystyrene resin, and an acrylic resin.

17. A parallel-filter connector to be connected to a body of a cell culture vessel, wherein the connector comprises an internal cavity having mutually communicating three openings in a manner that one of the three openings is configured to be connected to a corresponding opening provided in the body of the cell culture vessel, and the other two openings:
(1) respectively hold one of two types of filters in a manner that each of the filter transversely partitions the internal cavity and outside;
(2) are configured so that one of the openings is installed with one of two types of filters so as to transversely partition the internal cavity and outside, and the other opening is capable of being connected to a corresponding opening of a filter unit defining a flow pass running therethrough and holding the other of the two types of filters so as to transversely partition the flow pass; or
(3) are configured so that each of the other two openings is capable of being connected to a corresponding opening of each of two filter units, the filter units defining a flow pass running therethrough and holding one of two types of filters in a manner that the filters transversely partition the flow pass.

18. The parallel-filter connector according to claim 17, wherein the parallel-filter connector is provided with, at the opening to be connected to an opening of a body of a cell culture vessel, a part having a male screw thread, a part having a female screw thread, a male tapered fitting surface, or female tapered fitting surface, so as to fit to the corresponding opening of a body of a cell culture vessel.

19. The parallel-filter connector according to claim 17 or 18, wherein the parallel-filter connector is in the form of a generally branched tube having the three opening at each of the terminuses thereof.

20. The parallel-filter connector according to any one of claims 17 to 19, wherein one of the two types of filters is a membrane filter, and the other is a depth filter.

21. The parallel-filter connector according to claim 20, wherein a thickness of the membrane filter is 100 to 200 µm, and a thickness of the depth filter is 210 to 800 µm.

22. The parallel-filter connector according to claim 21, wherein a thickness of the membrane filter is 120 to 180 µm, and a thickness of the depth filter is 230 to 770 µm.

23. The parallel-filter connector according to any one of claims 17 to 22, wherein the filter unit is connected to one or two of the three openings.

24. A kit of (1) or (2) below for combining the parallel-filter connector according to claim 23:
(1) a kit including a parallel-filter connector installed with one of the two types of filters, and a filter unit installed with the other of the two types of filters;
(2) a kit including a parallel-filter connector, a filter unit installed with one of the two types of filters, and a filter unit installed with the other of the two types of filters.
